(19) **Europäisches Patentamt**

**European Patent Office**

**Office européen des brevets**

(11) **EP 1 671 629 A1**

(12) **EUROPEAN PATENT APPLICATION**
published in accordance with Art. 158(3) EPC

(43) Date of publication:
**21.06.2006 Bulletin 2006/25**

(51) Int Cl.:
*A61K 31/19* (1985.01)   *A61K 31/191* (2000.01)
*A61K 31/198* (2000.01)   *A61K 31/194* (2000.01)
*A61K 31/192* (2000.01)   *A61K 31/375* (1985.01)
*A61K 8/18* (0000.00)   *A61P 1/02* (2000.01)
*A61P 43/00* (2000.01)   *A61P 31/04* (2000.01)
*A61P 37/04* (2000.01)   *A61P 17/00* (2000.01)
*A61P 17/02* (2000.01)   *A61P 37/08* (2000.01)
*A61P 17/16* (2000.01)   *A61P 17/10* (2000.01)
*A61P 27/02* (2000.01)   *A61P 31/16* (2000.01)
*A61P 11/02* (2000.01)   *A61P 31/22* (2000.01)
*A61P 31/10* (2000.01)

(21) Application number: 04773408.2

(22) Date of filing: 17.09.2004

(86) International application number:
**PCT/JP2004/014024**

(87) International publication number:
**WO 2005/027893 (31.03.2005 Gazette 2005/13)**

(84) Designated Contracting States:
**AT BE BG CH CY CZ DE DK EE ES FI FR GB GR HU IE IT LI LU MC NL PL PT RO SE SI SK TR**

(30) Priority: **19.09.2003 JP 2003328704**

(71) Applicant: **Otsuka Pharmaceutical Company, Limited**
**Tokyo 101-8535 (JP)**

(72) Inventors:
• **IGARASHI, Sachiyo**
  **Otsu-shi, Shiga 520-0054 (JP)**
• **TANAKA, Hideo**
  **Kyoto-shi,**
  **Kyoto 606-8116 (JP)**

• **TAKASU, Osamu**
  **Himeji-shi,**
  **Hyogo 672-8070 (JP)**
• **SHINOHARA, Shigeo**
  **Kyoto-shi,**
  **Kyoto 606-8275 (JP)**
• **TANAKA, Masahiko**
  **5200246 (JP)**
• **YAGO, Misato**
  **Kyoto-shi,**
  **Kyoto 607-8011 (JP)**

(74) Representative: **HOFFMANN EITLE**
**Patent- und Rechtsanwälte**
**Arabellastrasse 4**
**81925 München (DE)**

(54) **HUMAN BETA-DEFENSIN SECRETION PROMOTER**

(57)    The present invention provides a human β-defensin secretion promoter that can be used in various forms, such as external preparation, internal preparation, food, etc., and promotes human β-defensin secretion.

An organic acid has an effect of promoting the secretion of human β-defensin, particularly human β-defensin-2, and therefore the organic acid is used as an active ingredient of a human β-defensin secretion promoter. Furthermore, by adding the human β-defensin secretion promoter to external preparations, internal preparations, or foods, a human β-defensin secretion promotion effect can be imparted thereto.

Fig. 2

EP 1 671 629 A1

**Description**

TECHNICAL FIELD

The present invention relates to a β-defensin secretion promoter that is capable of promoting secretion of β-defensin. The present invention also relates to a method for promoting secretion of β-defensin.

BACKGROUND OF THE INVENTION

It is commonly known that plants, insects, amphibians, mammals, etc., inherently have antimicrobials (antibacterial peptides) in vivo as a biophylaxis mechanism. These antimicrobials are called natural immunity and locally achieve phylactic function in vivo. Defensin, which is known as one of the human antibacterial peptides, exhibits antibacterial activities against bacteria, fungi, protozoa, viruses, etc., and is involved in various biophylaxis mechanisms in vivo.

β-defensin is known as a defensin that is expressed on the mucosal epithelia of the skin, lungs, trachea, kidneys, genitals, etc. To date, six types of human β-defensins (i.e., human β-defensin-1, human β-defensin- 2 , human β-defensin-3 , human β-defensin- 4 , human β-defensin- 5 and human β-defensin-6) have been isolated and their structures have been identified. In particular, human β-defensin-2 has been shown to have characteristics such that its expression is induced by bacterial infections or inflammatory cytokine stimulation that is dominantly expressed on skin, lungs, trachea and oral mucosa (Tetsuji Tomita, "Defensins as a mechanism of host defense and innate immunity", Japanese Journal of Geriatrics 38 (4) : 440-443, 2001). It is suggested that human β-defensin-2 has a close relation with pneumonia and other trachea infections and inflammations.

In recent years, it has also been reported that β-defensins relate to not only local phylaxis but also to acquired immunity by chemotactic migration of dendritic cells, T lymphocytes, monocytes, etc. (Yang, D., Chertov, O., Bykovskaia, S.N. et al., "β-Defensins: linking innate and Adaptive immunity through dendritic and T cell CCR6", Science, 286: 525, 1999.; Territo, M.C., Ganz, T., Selsted, M.E. et al., "Monocyto-chemotactic activity of defensins from human neutrophils", J. Clin. Invest., 84: 2017, 1989.; Lillard, J.W., Jr., Boyaka, P.N., Chertov O. et al., "Mechanisms for induction of acquired host immunity by neutrophil peptide defensins", Proc. Natl. Acad. Sci. U.S.A., 96: 651,1999.; Isao Nagaoka et al., "Phylaxis of Defensin and its role in immune response", Rinsho Men'eki, 33: 577, 2000.).

It is commonly known that inflammatory cytokine and lipopolysaccharide have the secretion promoting effect of human β-defensins. However, the production and secretion mechanisms of human β-defensin have not been satisfactorily elucidated, and no means for promoting the secretion of human β-defensin have been found other than using the substances mentioned above.

SUMMARY OF THE INVENTION

An object of the present invention is to provide a β-defensin secretion promoter that can promote secretion of human β-defensin. Furthermore, the present invention provides a human β-defensin secretion promoter that can be used in various forms, such as external preparations, internal preparations, foods, etc.

The present invention also provides a method for effectively promoting human β-defensin secretion.

The present inventors conducted extensive research to solve the above problems and found that an organic acid is effective in promoting human β-defensin secretion. The present invention has been accomplished based on such findings and by conducting further extensive research.

In other words, the present invention provides a human β-defensin secretion promoter such as that described below:

Item 1. A human β-defensin secretion promoter comprising an organic acid as an active ingredient.

Item 2. A human β-defensin secretion promoter according to Claim 1, wherein the organic acid is at least one member selected from the group consisting of fumaric acid, malic acid, citric acid, ascorbic acid, lactic acid, acetic acid, adipic acid, tartaric acid, cinnamic acid, glutamic acid and succinic acid.

Item 3. A human β-defensin secretion promoter according to Claim 1, wherein the organic acid is at least one member selected from the group consisting of fumaric acid, malic acid, citric acid and tartaric acid.

Item 4. A human β-defensin secretion promoter according to Claim 1, wherein the human β-defensin subjected to secretion promotion is human β-defensin-2.

Item 5. A human β-defensin secretion promoter according to Item 1, which is used in the labial region or the oral cavity.

Item 6. An external-use composition for promoting human β-defensin secretion comprising a human β-defensin secretion promoter of any one of Items 1 to 4.

Item 7. An internal-use composition for promoting human β-defensin secretion comprising a human β-defensin secretion promoter of any one of Items 1 to 4.

Item 8. A food for promoting human β-defensin secretion comprising a human β-defensin secretion promoter of any one of Items 1 to 4.

The present invention provides a method for promoting human β-defensin secretion as described below:

Item 9. A method for promoting human β-defensin secretion comprising the step of administering or allowing a subject to intake an effective amount of organic acid.
Item 10. A method for promoting human β-defensin secretion according to Item 9, wherein the organic acid is at least one member selected from the group consisting of fumaric acid, malic acid, citric acid, ascorbic acid, lactic acid, acetic acid, adipic acid, tartaric acid, cinnamic acid, glutamic acid and succinic acid.
Item 11. A method for promoting human β-defensin secretion according to Item 9, wherein the organic acid is at least one member selected from the group consisting of fumaric acid, malic acid, citric acid and tartaric acid.
Item 12. A method for promoting human β-defensin secretion according to Item 9, wherein the human β-defensin is human β-defensin-2.
Item 13. A method for promoting human β-defensin secretion according to Item 9, wherein secretion of human β-defensin is promoted in the labial region or the oral cavity.
Item 14. A method for promoting human β-defensin secretion according to Item 9, wherein the human β-defensin secretion promoter of any one of Items 1 to 5 is used as the organic acid.
Item 15. A method for promoting human β-defensin secretion according to Item 9, wherein an internal-use composition of Item 6 is used as the organic acid.
Item 16. A method for promoting human β-defensin secretion according to Item 9, wherein a food of in Item 8 is used as the organic acid.
Item 17. A method for promoting human β-defensin secretion according to Item 9, wherein an external-use composition of Item 6 is used as the organic acid.

Furthermore, the present invention provides use of an organic acid in the following manner:

Item 18. Use of an organic acid for preparing a human β-defensin secretion promoter.
Item 19. Use of an organic acid for preparing an internal-use composition for promoting human β-defensin secretion.
Item 20. Use of an organic acid for preparing foods for promoting human β-defensin secretion.
Item 21. Use of an organic acid for preparing an external-use composition for promoting human β-defensin secretion.
Item 22. Use of an organic acid for promoting human β-defensin secretion.

BRIEF DESCRIPTION OF DRAWINGS

Fig. 1 shows the promoted secretion amounts of human β-defensin-2 (ng/min) in saliva when each organic acid was applied to the oral cavity in Test Example 1.
Fig. 2 shows the promoted secretion amounts of human β-defensin-2 (ng/min) in saliva when each organic acid was applied to the oral cavity in Test Example 2.
Fig. 3 shows the promoted secretion amounts of human β-defensin-2 (ng/min) in saliva when various amounts of malic acids were applied to the oral cavity in Test Example 3.

MODE FOR CARRYING OUT THE INVENTION

(1) Human β-defensin secretion promoter

Human β-defensin-1, human β-defensin-2, human β-defensin-3, human β-defensin-4, human β-defensin-5 and human β-defensin-6 are included in the human β-defensins for which secretion is promoted by the human β-defensin secretion promoter of the present invention. Among these, human β-defensin-2 is the most desirable object because its secretion is promoted in an efficient manner.
The active ingredient of the β-defensin secretion promoter of the present invention is an organic acid. There is no restriction on the organic acids used in the present invention as long as they are pharmacologically, cosmetically and food-hygienically acceptable. Examples of the organic acids used in the present invention include formic acid, acetic acid, propionic acid, butyric acid, valeric acid, caproic acid, caprylic acid, caprin acid, lauric acid, myristic acid, palmitic acid, stearic acid , acrylic acid, propionyl acid, cinnamic acid, caffeic acid and like monocarboxylic acids; oxalic acid, malonic acid, succinic acid, glutaric acid, adipic acid, pimelic acid, fumaric acid, maleic acid, phthalic acid, isophthalic acid, terephthalic acid and like dicarboxylic acids; glutamic acid, aspartic acid and like amino acids; glycolic acid, malic acid, tartaric acid, isotartaric acid, citric acid, isocitric acid, lactic acid, hydroxyacrylic acid, a-oxybutyric acid, glyceric acid, tartronic acid, salicylic acid, gallic acid, tropic acid, ascorbic acid, gluconic acid and like hydroxy acids; folic acid; pantothenic acid; nicotinic acid; and other organic acids of saccharide derivatives. Among these, fumaric acid, malic acid, citric acid, ascorbic acid, lactic acid, acetic acid, adipic acid, tartaric acid, cinnamic acid, glutamic acid and succinic acid are preferble, and fumaric acid, malic acid, citric acid and tartaric acid are particularly preferable. These organic acids may be used singly or in a combination of two or more. In the present invention, salts of the organic acids that are pharmacologically, cosmetically and hygienically acceptable may be used as an active ingredient instead of the above-mentioned organic acids or together with the above-mentioned organic acids.

Such organic acids are not necessarily purified, and any material may be used as an active ingredient of the human β-defensin secretion promoter of the present invention as long as at least one organic acid mentioned above is contained.

The human β-defensin secretion promoter of the present invention may consist of the above active ingredients or comprise base materials, carriers, additives, etc., that are food hygienically, pharmacologically and cosmetically acceptable, together with the active ingredient.

The object to which the human β-defensin secretion promoter of the present invention can be applied is not limited and may be any body parts or tissues in vivo. From the viewpoint of achieving excellent promotion of human β-defensin secretion, inside the nasal cavity, the anal region, inside the respiratory tract, the labial region, inside the oral cavity, the pharynx, the eye, the genitals, the skin, inside a digestive organ, etc., are preferable, and the mucocutaneous junction and mucosal epithelium of the above-mentioned parts are particularly preferable. Because human β-defensin secretion promotion effects observed in the labial region and inside the oral cavity are excellent, the labial region and inside the oral cavity are the most preferable objects to which the human β-defensin secretion promoter of the present invention is applied.

The human β-defensin secretion promoter of the present invention can be used as a food or an internal-use composition for promoting human β-defensin secretion by adding it to a food, an internal-use medicine, an internal-use quasi-drug, etc. The human β-defensin secretion promoter of the present invention can also be used as an external-use composition for promoting human β-defensin secretion by adding it to a medicine for external use, a quasi-drug for external use, a cosmetic, a medical product for external use (e.g., adhesive plaster), etc.

An external-use composition for promoting human β-defensin secretion comprising the human β-defensin secretion promoter of the present invention can be prepared by adding pharmacologically or cosmetically acceptable base materials and/or carriers to the above-mentioned active ingredients and preparing it into a desirable form. Furthermore, various kinds of additives, such as a surfactant, a coloring agent (dye, pigment), a fragrance, a preservative, a germicide (antimicrobial agent), a thickener, an antioxidant, sequestering agent, a refreshing agent, a deodorant, a pH adjuster, etc., can be added as long as they do not impair the effects of the present invention. If necessary, it is also possible to add known pharmaceutically effective ingredients used in external preparations, such as a humectant, an ultraviolet absorber, a ultraviolet distraction agent, vitamins, a plant extract, an astringent, an antiinflammatory agent (antiphlogistic agent), a whitening agent, a cell activator, vasodilator, a circulation accelerator, a skin hyperergasia agent, an antiallergic agent, an antihistamine, an antibiotic, etc.

The form of the external-use composition is not limited as long as it can be applied to the skin or mucous membrane. For example, it can be formed into a liquid, a milky lotion, a powder, a suspension, a cream, an ointment, a mousse, a gel, a jelly, a paste, a jell, a stick, an aerosol, a spray, a liniment, a pack, a patch, etc. The form of the external-use composition can be suitably selected depending on the part of the body to which it will be applied.

As long as it can exhibit human β-defensin secretion promoting effects, the above-explained external-use composition may be formed into an external-use composition also having effects other than the promotion of human β-defensin secretion. Specifically, the external-use composition may be formed into a wound-healing agent, a bed-bath agent, a cleaning agent (e.g., facial wash, cleansing cream, body soap, etc.), a basic skin care product (e.g., milky lotion, cream, lotion, oil, pack, etc.), a dentifrice, a mouthwash, a mouth deodorant, an intraoral patch, etc. An intraoral patch, dentifrice, mouthwash, and mouth deodorant are particularly preferable from the viewpoint of promoting human β-defensin secretion when applied in the oral cavity.

The above-explained external-use composition may be in the form of an enema agent, an ophthalmic solution, a collunarium, etc.

When a volatile organic acid is used as an active ingredient of the human β-defensin secretion promoter, by vaporizing the active ingredient and inhaling it, such a human β-defensin secretion promoter can be applied to the nasal cavity, the oral cavity and the bronchus. An example of using the human β-defensin secretion promoter in such a form includes an example in which an aqueous solution comprising the active ingredient is heated, etc., and the obtained vapor is inhaled. The above-explained external-use composition may also be made into a form that is used by vaporizing the active ingredient. When the above-explained external-use composition is made into such a form, the external-use composition may be used as an aromatic liquid by adding a fragrance component. Acetic acid, propionic acid, formic acid, butyric acid, valeric acid, etc., are examples of volatile organic acids used as an active ingredient of the human β-defensin secretion promoter.

The proportion of the human β-defensin secretion promoter in the above-explained external-use composition for promoting human β-defensin secretion may be suitably selected depending on the form of the composition, the type of the active ingredient, the age and gender of the subject, the expected effects, etc. For example, the proportion of the total weight of the active ingredient in the human β-defensin secretion promoter may be 0.001 wt% or greater, preferably 0.005 to 99 wt%, and more preferably 0.01 to 99 wt% relative to the total weight of the external-use composition.

The human β-defensin secretion promoter of the present invention can exhibit β-defensin secretion promotion effects when applied to skin or a mucous membrane. The dosage and number of applications of the human β-defensin secretion promoter may be suitably selected depending on the type and concentration of the active ingredient, age and gender of the subject, application form, expected effects, etc. For example, 0.1 mg or more, or preferably 0.5 to 10000 mg of the active ingredient of the human β-defensin secretion promoter may be applied to the skin or a mucous membrane about 1 to 10 times per day.

Food for promoting human β-defensin secretion comprising the human β-defensin secretion promoter of the present invention can be prepared by adding the above-described active ingredient to food (including beverages) as one of the ingredients during the preparation process of the food. The food for promoting human β-defensin secretion may contain various food additives that are hygienically acceptable as a food product.

Foods for specified health use, supplements, foods for the sick, etc., are examples of the above-mentioned food. More specifically, beverages (carbonated beverages, soft drinks, milk beverages, alcoholic beverages, juices, teas, energy drinks, etc.), powdered beverages (powdered juices, powdered soups, etc.), snacks (gum, candies, cookies, gummy candies, rice crackers, biscuits, jelly, caramels, sweet tarts, edible sheets, edible films, troches, etc.), mouth deodorants (gum, candies, gummy candies, edible sheets, edible films, troches, etc.), milk products (cheese, yogurt, etc.), bread, noodles, cereal, seasonings (sauce, dressing, etc.), etc. Gum, gummy candies, sweet tarts, candies, edible sheets and edible films are preferable from the viewpoint of promoting human β-defensin secretion in the oral cavity.

The proportion of the human β-defensin secretion promoter contained in the above-described foods for promoting human β-defensin secretion may be suitably selected depending on the type of the active ingredient, the age and gender of the subject, the expected effects, etc. For example, the proportion of the total weight of the active ingredient in the human β-defensin secretion promoter is 0.01 wt% or more, preferably 0.02 to 100 wt%, and more preferably 0.05 to 100 wt% relative to the total weight of the food.

The daily intake amount of a food for promoting human β-defensin secretion may be suitably selected depending on the form of the composition, the type and concentration of the active ingredient, the age and gender of the subject, the form of the food, the extent of the expected effects, etc., so that an amount effective for promoting human β-defensin secretion is ingested. For example, the amount of food ingested per day is selected so that 0.1 mg or more, and preferably 0.5 to 10000 mg or more of the active ingredient of the human β-defensin secretion promoter is contained.

Internal-use pharmaceutical compositions or internal-use quasi-drug compositions for promoting human β-defensin secretion that comprise the human β-defensin secretion promoter of the present invention may be prepared by adding a pharmacologically acceptable base material and/or carrier to the active ingredient and forming it into a desired form. Furthermore, pharmacologically acceptable additives such as a binder, disintegrator, lubricant, humectant, buffer, preservative, fragrance, etc., may be optionally added.

The forms of internal-use pharmaceutical compositions and internal-use quasi-drug compositions are not limited, but preferably they are soluble on a mucous membrane, in particular in the oral cavity. A film, troche, chewable tablet, pulvis, tablet, granule, capsule, syrup, etc., are examples of such forms. From the viewpoint of being easily soluble in the oral cavity, a film, troche and chewable tablet are preferable.

The proportion of the human β-defensin secretion promoter relative to an internal-use pharmaceutical composition and internal-use quasi-drug composition may be suitably selected depending on the type of active ingredient, the form of the composition, the age and gender of the subject, the expected effect, etc. For example, the total weight of the active ingredient of the human β-defensin secretion promoter may be 0.01 wt% or more, preferably 0.02 to 99 wt%, and more preferably 0.05 to 99 wt% relative to the total weight of the internal-use pharmaceutical composition or internal-use quasi-drug composition.

The daily ingestion amount of the internal-use pharmaceutical composition or internal-use quasi-drug composition may be suitably selected depending on the type and concentration of the active ingredient, the age and gender of the subject, the form of the composition, the administration method, the extent of the expected effect, etc., and cannot be uniformly specified but the amount to be ingested may be freely selected provided that it is effective for promoting human β-defensin secretion. For example, the dosage thereof may be selected so that 0.1 mg or more, and preferably 0.5 to 10000 mg of the active ingredient of the human β-defensin secretion promoter is contained, and such an amount of the internal-use pharmaceutical composition or internal-use quasi-drug composition may be administered 1 to 10 times per day.

As described above, an organic acid can promote human β-defensin secretion. Therefore, the present invention also provides the use of an organic acid in preparing the human β-defensin secretion promoter. Furthermore, the present invention provides the use of an organic acid in preparing an external-use composition and/or internal-use composition for promoting human β-defensin secretion.

(2) Method for promoting human β-defensin secretion

As described above, an organic acid can effectively promote human β-defensin secretion. Therefore, the present invention provides a method for promoting human β-defensin secretion using an organic acid.

The method for promoting human β-defensin secretion of the present invention can be conducted by applying an organic acid in a form of an external-use composition to the part of the body in which human β-defensin secretion is to be promoted. The method for promoting human β-defensin secretion of the present invention can also be conducted by administering or allowing a subject to take an organic acid in the form of an internal-use composition. The method of the present invention is conducted by using preferably a "human β-defensin secretion promoter" mentioned in Section (1) described above, and more preferably an external-use composition, an internal-use composition or a food for promoting human β-defensin secretion.

In the method of the present invention, the β-defensin for which secretion is to be promoted, the organic acid used, the dosage and number of administrations of the organic acid, the part (or tissue) in the body in which β-defensin secretion is to be promoted, etc., are the same as those mentioned in Section (1) described above.

By using an organic acid, human β-defensin secretion can be effectively promoted. Therefore, the present invention also provides the use of an organic acid for such promotion.

(3) Method for promoting human β-defensin secretion -2

The present inventors also found that human β-defensin secretion can be promoted in the oral cavity by making the oral cavity temporarily acidic. Based on this finding, the present invention also provides a method for promoting human β-defensin secretion in the oral cavity. To promote human β-defensin secretion by employing such a method, the oral cavity is made temporarily acidic, specifically, with the pH in the oral cavity being not greater than 7, and preferably not greater than 6.2. The duration in which the oral cavity is made acidic may vary as long as it is an effective duration for promoting human β-defensin secretion. In this method, the oral cavity can be made acidic by simply administering the above-mentioned organic acid into the oral cavity. An example of a preferred embodiment of such a method is the administration of the previously-mentioned human β-defensin secretion promoter into the oral cavity. Such a method can keep the oral cavity healthy, making the method effective for preventing or alleviating periodontitis, stomatitis, dental caries, halitosis, etc.

There is no limitation to the pH condition on the skin or mucous membrane in the use of the β-defensin secretion promoter descried in Section (1) above, or in the method for promoting β-defensin secretion descried in Section (2) above.

EXAMPLES

The present invention is explained below with reference to Examples and Test Examples; however, the scope of the present invention is not limited to these Examples. Note that human β-defensin is sometimes referred to as hBD-2.

Example 1 Gum

Gum having the following formulation was prepared in a routine manner.

| Ingredients | Amount (wt%) |
| --- | --- |
| Powdered lime | 5.0 |
| (Powdered lime comprising 75 wt% citric acid) Gum base (principal ingredient: vinyl acetate) | 22.0 |
| Xylitol | 35.0 |
| Maltitol | 27.0 |
| Flavor | q.s. |
| Total | 100.0 wt% |

Example 2 Sweet tarts

Sweet tarts having the following formulation were prepared in a routine manner.

| Ingredients | Amount (wt%) |
|---|---|
| Malic acid | 7.5 |
| Sodium bicarbonate | 7.5 |
| Xylitol | 40.0 |
| Maltitol | 30.0 |
| Cornstarch | 15.0 |
| Total | 100.0 wt% |

Example 3 Mouth deodorant (liquid)

Mouth deodorant (liquid) having the following formulation was prepared in a routine manner.

| Ingredients | Amount (wt%) |
|---|---|
| Lactic acid | 0.2 |
| 1-Menthol | 1.0 |
| Ethanol | 40.0 |
| Glycerol | 20.0 |
| Polyglyceryl-10 laurate | 0.5 |
| Perfumes | 0.1 |
| Purified water | Balance |
| Total | 100.0 wt% |

Example 4 Mouth deodorant (troche)

Mouth deodorant (troche) having the following formulation was prepared in a routine manner.

| Ingredients | Amount (wt%) |
|---|---|
| Tartaric acid | 45 |
| 1-Menthol | 1 |
| Sodium bicarbonate | 24 |
| Lactose | 25 |
| Sucrose esters of fatty acids | 3 |
| Flavoring agents | 1 |
| Sweetening agents | 1 |
| Total | 100 wt% |

Example 5 Food (troche)

Food (troche) having the following formulation was prepared in a routine manner.

| Ingredients | Amount (wt%) |
|---|---|
| Ascorbic acid | 5.0 |
| Lactose | 85.5 |
| Xylitol | 5.0 |
| Sucrose esters of fatty acids | 4.0 |
| Flavoring agents | 0.5 |
| Total | 100.0 wt% |

Example 6 Liquid dentifrice

Liquid dentifrice having the following formulation was prepared in a routine manner.

| Ingredients | Amount (wt%) |
| --- | --- |
| Fumaric acid | 45 |
| 1-Menthol | 1 |
| Sodium bicarbonate | 24 |
| Lactose | 25 |
| Sucrose esters of fatty acids | 3 |
| Flavoring agents | 1 |
| Sweetening agents | 1 |
| Total | 100.00 wt% |

Example 7 Aromatic liquid

An aromatic liquid having the following formulation was prepared in a routine manner.

| Ingredients | Amount (wt%) |
| --- | --- |
| Purified water | 54 |
| Absolute ethanol | 40 |
| Lemon extract | 4 |
| Grapefruit extract | 1 |
| Acetic acid | 1 |
| Fragrance | q.s. |
| Total | 100 wt% |

Example 8 Collunarium

A collunarium having the following formulation was prepared in a routine manner.

| Ingredients | Amount (wt%) |
| --- | --- |
| Sodium chloride | 0.9 |
| Citric acid | 1.0 |
| pH adjuster | q.s. |
| Purified water | Balance |
| Total | 100.0 wt% |

Example 9 Ophthalmic solution

An ophthalmic solution having the following formulation was prepared in a routine manner.

| Ingredients | Amount (wt%) |
| --- | --- |
| Sodium chloride | 0.9 |
| Potassium chloride | 0.1 |
| Flavin adenine dinucleotide | 0.05 |
| Sodium hyaluronate | 0.1 |
| Citric acid | 1.0 |
| pH adjuster | q.s. |
| Purified water | Balance |
| Total | 100.00 wt% |

Test Example 1 β-defensin secretion promotion confirmatory test-1

To evaluate β-defensin secretion promotion effects of various organic acids, the following tests were conducted using one healthy adult as a subject.

Saliva secreted for 3 minutes from a rested subject was collected by a spitting method (hereunder, this is referred to as the control). Thereafter, 50 mg of fumaric acid was placed in the oral cavity, held for one minute, and then collected together with the secreted saliva. The concentration of β-defensin-2 in the collected saliva was measured by ELISA, and the amount of β-defensin-2 secreted over one minute due to the organic acid was calculated based on the formula below. Note that the saliva amount (ml) in the formula below was calculated based on the weight of the collected saliva (g) assuming 1 g of saliva = 1 ml of saliva. The same tests were conducted using ascorbic acid, citric acid, malic acid, lactic acid, adipic acid, acetic acid, and tartaric acid instead of fumaric acid. When lactic acid and acetic acid were used, the above tests were conducted by adding water to 50 mg of the organic acid so that the total weight was 1 g and placing it in the oral cavity.

## Formula

```
[Amount of hBD-2 secretated over 1 minute (ng/min)]

= {[Concentration of hBD-2 in saliva (ng/ml)] x [saliva (ml)]}/[Duration of  saliva
collection (min)]

* Duration of saliva collection in the control was 3 minutes and after administration of
organic acid was 1 minute
```

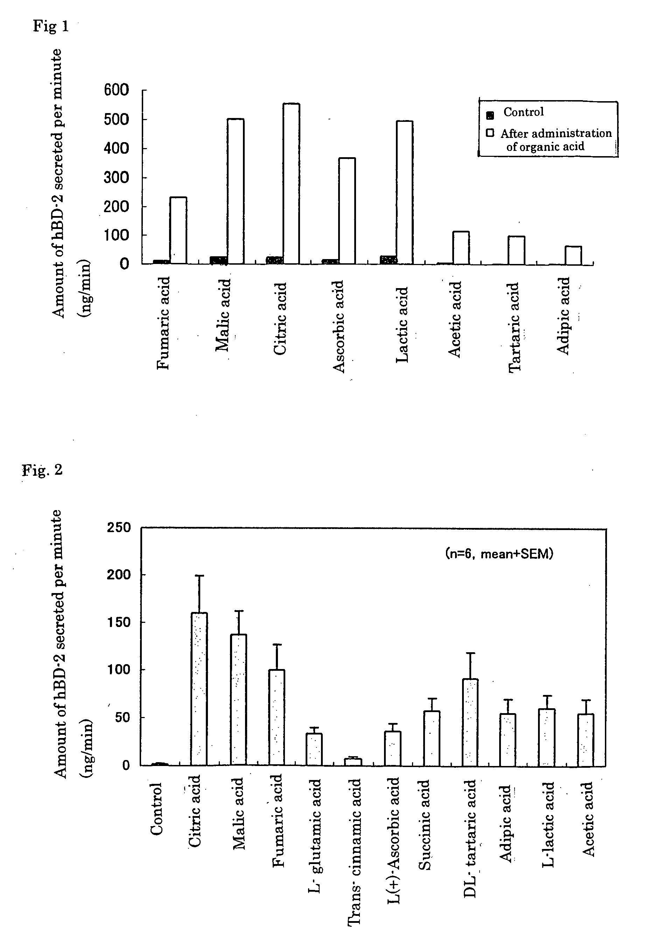

Fig. 1 shows the results. By applying organic acid in the oral cavity, the amount of the β-defensin-2 secreted in saliva is clearly increased. From this result, it was confirmed that the organic acids promote secretion of β-defensin-2 in vivo, and are particularly excellent in promoting secretion of β-defensin-2 in the oral cavity.

Test Example 2 β-defensin secretion promotion confirmatory test-2

To evaluate β-defensin secretion promotion effects of various organic acids, the following tests were conducted using 6 healthy adults as subjects. Citric acid, malic acid, fumaric acid, L-glutamic acid, trans-cinnamic acid, L(+)-ascorbic acid, succinic acid, DL-tartaric acid, adipic acid, L-lactic acid, and acetic acid were used as the organic acid.

Saliva secreted for 3 minutes from rested subjects was collected by a spitting method (hereunder, this is referred to as the control). Thereafter, 50 mg of organic acid was placed in the oral cavity, held for one minute, and then collected together with the secreted saliva. The concentration of β-defensin-2 in the saliva was measured by ELISA, and the amount of β-defensin-2 secreted over one minute using the organic acid was calculated based on the formula below. Note that the saliva amount (ml) in the formula below was calculated based on the weight of the collected saliva (g) assuming 1 g of saliva = 1 ml of saliva. When lactic acid and acetic acid were used, the above tests were conducted by adding water to 50 mg of the organic acid so that the total weight was 1 g and placing it in the oral cavity.

## Formula

```
[Amount of hBD-2 secretated over 1 minute (ng/min)]

= {[Concentration of hBD-2 in saliva (ng/ml)] x [saliva (ml)]}/[Duration of  saliva
collection (min)]

* Duration of saliva collection in the control was 3 minutes and after administration of
organic acid was 1 minute
```

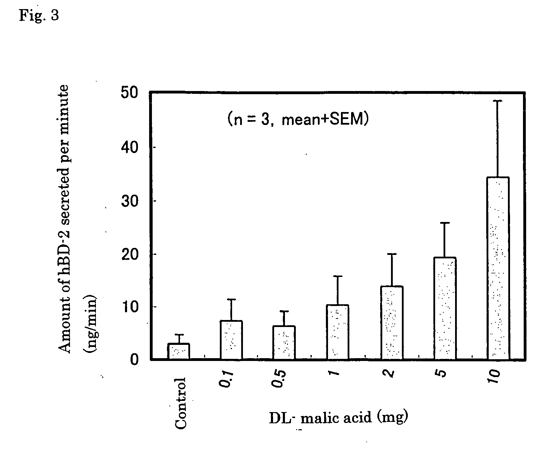

Fig. 2 shows the results. From this result, it was confirmed that the organic acids promote secretion of β-defensin-2 in vivo, and are particularly excellent in promoting secretion of β-defensin-2 in the oral cavity.

Test Example 3 β-defensin secretion promotion confirmatory test-3 (concentration dependency)

To evaluate β-defensin secretion promotion effects and concentration dependency of an organic acid, the following tests were conducted using 3 healthy adults as subjects.

Saliva secreted for 3 minutes from rested subjects was collected by a spitting method (hereunder, this is referred to as the control). Thereafter, 1 ml of 0.01 wt% malic acid (amount of malic acid ingested: 0.1 mg) was placed in the oral cavity, held for one minute, and then collected together with the secreted saliva. The concentration of β-defensin-2 in the saliva was measured by ELISA, and the amount of β-defensin-2 secreted over one minute using 0.1 mg of malic acid was calculated based on the formula below. Note that the saliva amount (ml) in the formula below was calculated based on the weight of the collected saliva (g) assuming 1 g of saliva = 1 ml of saliva. The same test was conducted, respectively, using 0.05, 0.1, 0.2, 0.5, or 1 wt% malic acid aqueous solution (amounts of ingested: 0.5, 1, 2, 5, and 10 mg).

## Formula

```
[Amount of hBD-2 secretated over 1 minute (ng/min)]

= {[Concentration of hBD-2 in saliva (ng/ml)] x [saliva (ml)]}/[Duration of  saliva
collection (min)]

 * Duration of saliva collection in the control was 3 minutes and after administration of
organic acid was 1 minute
```

Fig. 3 shows the results. As is clear from Fig. 3, the amount of β-defensin-2 secreted in saliva increases depending on the concentration of malic acid aqueous solution. From this result, it was confirmed that the human β-defensin-2 secretion promoting effect of the organic acid has concentration dependency, and that the organic acid has a human β-defensin-2 secretion induction effect even at the concentration of 0.01% (amount of ingested: 0.1 mg).

## INDUSTRIAL APPLICABILITY

It is known that human β-defensin has the following excellent properties:

(a) because human β-defensin itself has bactericidal action, an antibacterial effect can be achieved in a short time;
(b) because human β-defensin is secreted in vivo, excellent antibacterial effects can be achieved in parts of the body to which conventional antibacterial agents administered in external-use or internal-use forms are ineffective: and (c) human β-defensin allows dendritic cells or memory T lymphocytes to act chemotactically (i.e., affecting acquired immunity by mobilizing immunocompetent cells).

The human β-defensin secretion promoter of the present invention can promote secretion of β-defensin, in particular human β-defensin-2, at the application site. Therefore, the human β-defensin secretion promoter of the present invention can promote β-defensin secretion and enhance antibacterial and/or immunization effects in vivo, thus enhancing protective mechanisms in vivo. Therefore, the human β-defensin secretion promoter of the present invention is effective in the prevention and alleviation of symptoms caused by infection with bacteria, fungi, viruses, and protozoa. In particular, the human β-defensin secretion promoter of the present invention is effective in skin care, such as preventing secondary-infections caused by atopic dermatitis, pimples, halitosis, bad foot odor, secondary-infections after shaving or depilation, secondary-infections caused by diaper rash, secondary-infections caused by heat rash, secondary-infections caused by thermal burns, and infectible infections caused by use of steroids, etc. Furthermore, the human β-defensin secretion promoter of the present invention is usable for skincare treating rough skin, dry skin, hand roughness, etc., caused by infection with bacteria, fungi, viruses, and/or protozoa.

The human β-defensin secretion promoter of the present invention is expected to exhibit pharmacological effects based on its human β-defensin secretion promotion effects. Therefore, the human β-defensin secretion promoter of the present invention is effective for treating viral conjunctivitis, bacterial conjunctivitis, hordeolum, etc., when applied to the eye; for colds, influenza, etc., when applied to the nasal cavity and/or pharynx; for rhinitis, pyogenic rhinorrhea, sinusitis, etc., when applied to the nasal cavity; for periodontitis, stomatitis, dental caries, etc., when applied in the oral cavity; for herpes simplex virus, etc., when applied to the labial region; for preventing tinea pedis, treating impetigo, verrucas, molluscum contagiosum, chicken pox, genital herpes virus infection, candidiasis, herpes zoster, herpes simplex, pimples, atopic dermatitis, wounds, etc., when applied to the skin; and for preventing or treating diseases, such as gastritis, tumors, acquired immuno-deficiency syndrome, trypanosomiasis, etc., when applied to other parts of the body. Among these diseases, the human β-defensin secretion promoter of the present invention is effective for preventing and/or treating those caused by bacteria, fungi, viruses, protozoa, etc.

Furthermore, because the human β-defensin secretion promoter of the present invention achieves excellent β-defensin secretion promotion effects in the oral cavity and labial region, it is particularly effective for preventing and/or treating periodontitis, stomatitis, dental caries, halitosis, herpes simplex, etc. Therefore, the human β-defensin secretion promoter of the present invention is suitably used in oral care products that keep the oral cavity healthy.

**Claims**

1. A human β-defensin secretion promoter comprising an organic acid as an active ingredient.

2. A human β-defensin secretion promoter according to Claim 1, wherein the organic acid is at least one member selected from the group consisting of fumaric acid, malic acid, citric acid, ascorbic acid, lactic acid, acetic acid, adipic acid, tartaric acid, cinnamic acid, glutamic acid and succinic acid.

3. A human β-defensin secretion promoter according to Claim 1, wherein the organic acid is at least one member selected from the group consisting of fumaric acid, malic acid, citric acid and tartaric acid.

4. A human β-defensin secretion promoter according to Claim 1, wherein the human β-defensin subjected to secretion promotion is human β-defensin-2.

5. A human β-defensin secretion promoter according to Claim 1, which is used in the labial region or the oral cavity.

6. An external-use composition for promoting human β-defensin secretion comprising a human β-defensin secretion promoter of any one of Claims 1 to 4.

7. An internal-use composition for promoting human β-defensin secretion comprising a human β-defensin secretion promoter of any one of Claims 1 to 4.

8. A food for promoting human β-defensin secretion comprising a human β-defensin secretion promoter of any one of Claims 1 to 4.

9. A method for promoting human β-defensin secretion comprising the step of administering or allowing a subject to intake an effective amount of organic acid.

10. A method for promoting human β-defensin secretion according to Claim 9, wherein the organic acid is at least one member selected from the group consisting of fumaric acid, malic acid, citric acid, ascorbic acid, lactic acid, acetic acid, adipic acid, tartaric acid, cinnamic acid, glutamic acid and succinic acid.

11. A method for promoting human β-defensin secretion according to Claim 9, wherein the organic acid is at least one member selected from the group consisting of fumaric acid, malic acid, citric acid and tartaric acid.

12. A method for promoting human β-defensin secretion according to Claim 9, wherein the human β-defensin is human β-defensin-2.

13. A method for promoting human β-defensin secretion according to Claim 9, wherein secretion of human β-defensin is promoted in the labial region or the oral cavity.

14. A method for promoting human β-defensin secretion according to Claim 9, wherein the human β-defensin secretion promoter of any one of Claims 1 to 5 is used as the organic acid.

15. A method for promoting human β-defensin secretion according to Claim 9, wherein an internal-use composition of Claim 6 is used as the organic acid.

16. A method for promoting human β-defensin secretion according to Claim 9, wherein a food set forth in Claim 8 is used as the organic acid.

17. A method for promoting human β-defensin secretion according to Claim 9, wherein an external-use composition of Claim 6 is used as the organic acid.

18. Use of an organic acid for preparing a human β-defensin secretion promoter.

**19.** Use of an organic acid for preparing an internal-use composition for promoting human β-defensin secretion.

**20.** Use of an organic acid for preparing foods for promoting human β-defensin secretion.

**21.** Use of an organic acid for preparing an external-use composition for promoting human β-defensin secretion.

**22.** Use of an organic acid for promoting human β-defensin secretion.

Fig 1

Fig. 2

Fig. 3

<div align="center">

**INTERNATIONAL SEARCH REPORT**

</div>

| | International application No. |
|---|---|
| | PCT/JP2004/014024 |

| A. CLASSIFICATION OF SUBJECT MATTER |
|---|
| Int.Cl⁷ A61K31/19, 31/191, 31/198, 31/194, 31/192, 31/375, 7/16, A61P1/02, 43/00, 31/04, 37/04, 17/00, 17/02, 37/08, 17/16, 17/10, 27/02, 31/16, 11/02, 31/22, 31/10, 29/00, 11/00, 1/04, 35/00, 31/18, 33/00, A23L1/30 |

According to International Patent Classification (IPC) or to both national classification and IPC

| B. FIELDS SEARCHED |
|---|
| Minimum documentation searched (classification system followed by classification symbols) |
| Int.Cl⁷ A61K31/19-31/194, 31/198, 31/375 |

Documentation searched other than minimum documentation to the extent that such documents are included in the fields searched

Electronic data base consulted during the international search (name of data base and, where practicable, search terms used)
CAPLUS(STN), MEDLINE(STN), BIOSIS(STN), EMBASE(STN), WPI

C. DOCUMENTS CONSIDERED TO BE RELEVANT

| Category* | Citation of document, with indication, where appropriate, of the relevant passages | Relevant to claim No. |
|---|---|---|
| X | JP 2003-512325 A (The Procter & Gamble Co.), 02 April, 2003 (02.04.03), Claims 2, 7; page 11, line 28 to page 12, line 5 & WO 2001/028556 A2 | 1-4,18 |
| X | JP 3-294227 A (Daiichi Pharmaceutical Co., Ltd.), 25 December, 1991 (25.12.91), Page 1, right column, lines 18 to 20 (Family: none) | 1,2,4,5 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 December, 2004 (07.12.04) | 28 December, 2004 (28.12.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

| **INTERNATIONAL SEARCH REPORT** | International application No. |
|---|---|
| | PCT/JP2004/014024 |

**C (Continuation).  DOCUMENTS CONSIDERED TO BE RELEVANT**

| Category* | Citation of document, with indication, where appropriate, of the relevant passages | Relevant to claim No. |
|---|---|---|
| X | WO 2001/093857 A1  (PALLADIN HEALTHCARE INTERNATIONAL, LTD.), 13 December, 2001 (13.12.01), Claim 1; page 4, line 31 to page 5, line 21; page 10, lines 7 to 17; page 12, line 24 to page 13, line 27 & JP 2003-535131 A | 1-4,6,18,21 |
| X | JP 55-31078 A  (Sterling Drug Inc.), 05 March, 1980 (05.03.80), Full text & EP 8121 A1           & US 4767788 A | 1,6,18,21 |
| P,X | JP 2004-67660 A  (Coletica), 04 March, 2004 (04.03.04), Full text & FR 2843125 A1          & DE 10251709 A1 & GB 2391476 A | 1,6,18,21 |
| X | WO 2001/068085 A1  (GENAERA CORP.), 20 September, 2001 (20.09.01), Full text & AU 4572601 A | 1,5,7,18,19 |
| X | JP 2003-95938 A  (Masami MORIYAMA), 03 April, 2003 (03.04.03), Full text (Family: none) | 1,7,8,18-20 |
| P,X | JP 2004-175733 A  (Masami MORIYAMA), 24 June, 2004 (24.06.04), Full text (Family: none) | 1,7,18,19 |
| E,X | JP 2004-262924 A  (Masami MORIYAMA), 24 September, 2004 (24.09.04), Full text (Family: none) | 1,7,18,19 |
| X | JP 2003-116486 A  (Meiji Milk Products Co., Ltd.), 22 April, 2003 (22.04.03), Claims 1, 8 (Family: none) | 8,20 |
| E,X | JP 2004-261181 A  (International Flavor and Fragrances Inc.), 24 September, 2004 (24.09.04), Full text (Family: none) | 8,20 |

Form PCT/ISA/210 (continuation of second sheet) (January 2004)

<table>
<tr><td colspan="2" align="center">**INTERNATIONAL SEARCH REPORT**</td><td>International application No.<br>PCT/JP2004/014024</td></tr>
<tr><td colspan="3">C (Continuation). DOCUMENTS CONSIDERED TO BE RELEVANT</td></tr>
<tr><td>Category*</td><td>Citation of document, with indication, where appropriate, of the relevant passages</td><td>Relevant to claim No.</td></tr>
<tr><td align="center">A</td><td>JP 2003-262 A (Morinaga & Co., Ltd.),<br>07 January, 2003 (07.01.03),<br>Full text<br>(Family: none)</td><td>1-8,18-21</td></tr>
<tr><td align="center">A</td><td>JP 2002-114704 A (Morinaga & Co., Ltd.),<br>16 April, 2002 (16.04.02),<br>Full text<br>(Family: none)</td><td>1-8,18-21</td></tr>
<tr><td align="center">A</td><td>Tetsuji TOMITA et al., "Seitai Bogyo Kiko<br>Toshiteno Defensin", Japanese journal of<br>geriatrics, 2001 Nen, Vol.38, pages 440 to<br>443</td><td>1-8,18-21</td></tr>
</table>

Form PCT/ISA/210 (continuation of second sheet) (January 2004)

## INTERNATIONAL SEARCH REPORT

| | International application No. |
|---|---|
| | PCT/JP2004/014024 |

---

**Box No. II**     **Observations where certain claims were found unsearchable (Continuation of item 2 of first sheet)**

This international search report has not been established in respect of certain claims under Article 17(2)(a) for the following reasons:

1. ☒ Claims Nos.: 9-17, 22
   because they relate to subject matter not required to be searched by this Authority, namely:
   The inventions as set forth in claims 9 to 17 and 22 pertain to methods for treatment of the human body by therapy and thus relate to a subject matter which this International Searching Authority is not required, under the provisions of Article 17(2)(a)(i) of the PCT    (continued to extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III**     **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐   The additional search fees were accompanied by the applicant's protest.

                            ☐   No protest accompanied the payment of additional search fees.

---

Form PCT/ISA/210 (continuation of first sheet (2)) (January 2004)

**INTERNATIONAL SEARCH REPORT**

International application No.

PCT/JP2004/014024

Continuation of Box No.II-1 of continuation of first sheet(2)

and Rule 39.1(iv) of the Regulations under the PCT, to search.

(Scope of international search)

Although claims 1, 4 to 8 and 18 to 21 relate to agents containing an organic acid having a property of promoting the secretion of human β-defensin, agents relating to only small part of the claimed organic acids are disclosed in the meaning within PCT Article 5. Thus, it appears that claims 1, 4 to 8 and 18 to 21 are not sufficiently supported by the description in the meaning within PCT Article 6.

Such being the case, the international search was made on the organic acids specifically cited in the description.

Complete search was made on claims 2 and 3.

Form PCT/ISA/210 (extra sheet) (January 2004)